# EUROPÄISCHE PATENTSCHRIFT

(11) **EP 1 606 006 B1**
(45) Veröffentlichungstag und Bekanntmachung des Hinweises auf die Patenterteilung: **01.10.2008**
(21) Anmeldenummer: 04719376.8
(22) Anmeldetag: 11.03.2004
(51) Int. Cl.: A61M 25/06, A61M 29/00

(54) **EINFÜHRVORRICHTUNG ZUM EINFÜHREN EINES GEGENSTANDES IN EIN KÖRPERGEFÄSS**
INTRODUCTION DEVICE FOR INTRODUCING AN OBJECT INTO A VESSEL IN THE BODY
DISPOSITIF POUR INTRODUIRE UN OBJET DANS UN VAISSEAU DU CORPS

(30) Priorität: 21.03.2003 DE 20304533 U
(43) Veröffentlichungstag der Anmeldung: 21.12.2005
(73) Patentinhaber: Abiomed Europe GmbH, 52074 Aachen (DE)
(72) Erfinder: SIESS, Thorsten, 52146 Würselen-Bardenberg (DE); PENNERS, Josef, 52445 Titz (DE)
(74) Vertreter: Selting, Günther
(86) Internationale Anmeldenummer: PCT/EP2004/002487
(87) Internationale Veröffentlichungsnummer: WO 2004/082755

(56) Entgegenhaltungen:
- EP-A- 0 596 172
- WO-A-02/43791
- DE-U- 9 108 043

## Beschreibung

Die Erfindung betrifft eine Einfühnrorrichtung zum Einführen eines Gegenstandes in ein Körpergefäß, mit einer rohrförmigen Schleuse und einem die Schleuse tragenden Dilatator, der einen konischen Spitzenbereich aufweist und aus der Schleuse zurückziehbar ist.

Eine derartige Einführvorrichtung ist beschrieben in WO 02/43791 A1 (Impella). Sie kann beispielsweise dazu benutzt werden, eine intravasale Pumpe durch ein Blutgefäß hindurch vorzuschieben. Auf diese Weise kann eine Blutpumpe in ein Blutgefäß des Körpers eingebracht und darin bis zum Einsatzort vorgeschoben werden. Die Schleuse besteht aus einem relativ steifen Rohr, das am proximalen (patientenfernen) Ende mit einem hämostatischen Ventil versehen ist. Die Schleuse wird über einen Dilatator geschoben. Durch den Dilatator führt ein Führungsdraht hindurch, der zuvor mittels Seldinger-Technik durch die Haut in das Blutgefäß eingeführt wurde. Über dem Führungsdraht wird der Dilatator zusammen mit der darauf sitzenden Schleuse vorgeschoben, wobei der Dilatator mit seinem konischen Spitzenbereich den durch das Körpergewebe hindurchführenden engen Kanal aufweitet (dilatiert).

Eine Einführvorrichtung nach dem Oberbegriff des Patentanspruchs 1 ist beschrieben in DE 91 08 043 U. Diese Einführvorrichtung weist eine langgestreckte rohrförmige Außenhülse aus Kunststoff auf, die gegen einen in sie eingesteckten Dilatator axial verschiebbar ist. Der distale Endabschnitt des Dilatators weist eine Einkerbung auf und die distale Spitze der Außenhülse ist konisch verjüngt, wobei sie die Einkerbung bündig ausfüllt. Wenn der Dilatator in der Außenhülse vorgeschoben wird, wird der verjüngte Teil der Außenhülse gedehnt und nimmt den Durchmesser des proximalen Dilatatorschaftes an. Nachdem der Durchmesser der Außenhülse auf diese Weise vergrößert worden ist, kann der Dilatator aus der Hülse leicht herausgezogen werden.

Wenn bei einer Einführvorrichtung der Dilatator das Blutgefäß erreicht hat und die Schleuse bis an ihre endgültige Position im Blutgefäß vorgeschoben worden ist, wird der Dilatator aus der Schleuse zurückgezogen. Nach Entnahme des Dilatators, der die Schleuse und das Gefäßareal vorübergehend geradegerichtet hat, wird die Schleuse eine Biegung machen, die der jeweiligen Anatomie entspricht. Dabei besteht die Gefahr, dass die Schleuse abknickt, oder der kreisrunde Querschnitt somit oval aufgrund der Krümmung deformiert wird, mit der Folge, dass der Schleusenkanal für den einzuführenden Gegenstand blockiert wird. Eine deformierte dickwandige Schleuse kann nur unter Aufbringung großer Axialkräfte, wie sie durch einen Dilatator eingebracht werden können, zur erneuten Freigabe des avisierten kreisrunden Querschnitts der Schleuse veranlasst werden.

Wird nun statt des Dilatators der einzuführende Katheter verwendet, der beispielsweise eine intravasale Blutpumpe enthalten kann, so sind die axial und radial aufzubringenden Kräfte reduziert und reichen gegebenenfalls nicht aus, um die Deformation der dickwandigen Schleuse zu beheben.

Bei einem relativ großen Innendurchmesser der Schleuse von 12 F (F = French) entsprechend 4 mm, wird infolge der Wandstärke der Schleuse der Außendurchmesser der auf dem Dilatator sitzenden Schleuse mit ca. 14-15 F sehr groß. Je größer der Durchmesser des Körperkanals ist, durch den die Schleuse hindurchführt, umso größer ist die Gefahr des Austretens von Blut nach Entnahme der Schleuse. Andererseits - muss die Schleuse eine gewisse Steifigkeit haben, damit sie sich während des Einführvorganges nicht ziehharmonikaartig auf dem Dilatator aufstaut. Auch beim Herausziehen des Dilatators aus der Schleuse besteht die Gefahr der ziehharmonikaartigen Faltung der Schleuse.

Der Erfindung liegt die Aufgabe zugrunde, eine Einführvorrichtung für relativ großformatige Gegenstände zu schaffen, bei der der zu erzeugende Körperkanal einen möglichst geringen Durchmesser hat.

Die Lösung dieser Aufgabe erfolgt erfindungsgemäß mit den im Anspruch 1 angegebenen Merkmalen. Hiernach hat die Schleuse eine Wandstärke von maximal 0,06 mm und sie besteht aus einem harten Kunststoff.

Unter einem harten Kunststoff sind harte und zähe Kunststoffe zu verstehen, wie beispielsweise Polyamid und Polyester. Die Schleuse hat eine extrem geringe Wandstärke von maximal 0,06 mm und insbesondere von maximal 0,03 mm. Bei einem Innendurchmesser der Schleuse von 4 mm ist der Außendurchmesser nur geringfügig größer. Damit wird der Durchmesser des zu erzeugenden Körperkanals, durch den die Schleuse hindurchführt, so klein wie möglich.

Vorzugsweise ist vor dem Aufschieben der Schleuse auf den Dilatator der Innendurchmesser der Schleuse mindestens gleich groß wie der Außendurchmesser des Dilatators. Dies bedeutet, dass beide Durchmesser bis auf ein Hunderstel mm einander gleich sind, so dass die Schleuse im Passsitz auf dem Dilatator sitzt. Es kann auch vorgesehen sein, dass die Schleuse im Presssitz auf dem Dilatator sitzt. Hierbei ist ihr Innendurchmesser geringfügig kleiner als der Außendurchmesser des Dilatators. Durch den festen Sitz wird erreicht, dass kein Spalt zwischen Dilatator und Schleuse vorhanden ist. Auch hierdurch wird das ziehharmonikaartige Aufstauen (Aufschuppen) der Schleuse beim Vorschieben durch das Körpergewebe und beim Zurückziehen des Dilatators vermieden. Insbesondere durch das Merkmal des harten Kunststoffs, der sich radial nicht dehnen lässt, wird ein Aufschuppen vermieden.

Um das Herausziehen des Dilatators aus der Schleuse zu erleichtern, ist gemäß einer bevorzugten Ausgestaltung der Erfindung vorgesehen, dass der Dilatator und/oder die Schleuse ein reibungsarmes Gleitmaterial aufweist. Beispielsweise kann der Dilatator oder die Schleuse eine Beschichtung aus Teflon tragen. In jedem Fall sollte die Materialpaarung von Schleuse und Dilatator im Berührungsbereich einen geringen Reibungsbeiwert µ haben. Der Dilatator kann hierzu auch mit einem Gleitmaterial benetzt sein.

Gemäß einer bevorzugten Weiterbildung der Erfindung ist eine Anschlussvorrichtung zum Injizieren einer Druckflüssigkeit in die Schleuse vorgesehen. Die Anschlussvorrichtung befindet sich am proximalen (patientenfernen) Ende der Schleuse. Sie kann an eine Druckquelle angeschlossen werden, mit der ein Druckmedium in die Schleuse injiziert wird, so dass diese geringfügig von dem Dilatator abhebt. Das Abheben erfolgt nur um einen sehr geringen Betrag, um das Gleiten auf einem dünnen Flüssigkeitsfilm zu ermöglichen. Das Maß des Abhebens ist viel kleiner als die Wandstärke der Schleuse und insbesondere kleiner als 5 % der Wandstärke.

Gemäß einer bevorzugten Weiterbildung der Erfindung, die jedoch auch selbständige Bedeutung hat, ist vorgesehen, dass die Schleuse einen distalen Endabschnitt aufweist, der den konischen Spitzenbereich des Dilatators mindestens teilweise überdeckt. Der distale Endabschnitt bewirkt eine Mitnahme der Schleuse beim Vorschieben des Dilatators und verhindert eine ziehharmonikaartige Aufstauchung der Schleuse beim Hindurchschieben durch das aufzuweitende und eng anliegende Körpergewebe. Bei dieser Ausführungsform kann ein schmaler Ringspalt zwischen Dilatator und Schleuse bestehen, dessen Spaltbreite maximal 0,2 mm beträgt. Beim Zurückziehen des Dilatators wird eine Faltung der Schleuse vermieden.

Der Endabschnitt der Schleuse kann von dem übrigen Bereich der Schleuse durch eine Abreißlinie begrenzt werden. Hierbei ist vorzugsweise der Endabschnitt haftend mit dem Spitzenbereich des Dilatators verbunden. Da infolge des zunächst in der Schleuse befindlichen Dilatators das Gewebe und der Gefäßapparat geradegerichtet worden sind, besteht nach Entnahme des Dilatators ebenfalls die Gefahr, dass sich die Schleuse an die anatomischen Krümmungen anpasst. Folglich werden auch hier Krümmungen vorliegen, die bei einer sehr dünnwandigen Schleuse automatisch zu Knicken führen.

Diese Knicke können aber im Gegensatz zu dickwandigen Schleusen leicht durch den einzuführenden Katheter geradegerichtet werden, da es sich hier um geringste Wandstärken handelt. Voraussetzung ist allerdings, dass der einzuführende Katheter über eine runde (sphärische) Spitze verfügt. So kann verhindert werden, dass beim Geraderichten der geknickten Schleuse die dünne Wand penetriert wird. Die Schleuse wirkt somit als Führung und ein auftretender Knick der Schleuse kann temporär beseitigt werden, bis der Katheter die Schleuse passiert hat.

Im Folgenden werden unter Bezugnahme auf die Zeichnungen Ausführungsbeispiele der Erfindung näher erläutert.

Es zeigen:
- Fig. 1: einen Längsschnitt durch das distale Ende der Einführvorrichtung,
- Fig. 2: die Einführvorrichtung während des Zurückziehens des Dilatators,
- Fig. 3: einen schematischen Längsschnitt der gesamten Einführvorrichtung mit einer Anschlussvorrichtung am proximalen Ende,
- Fig. 4: ein weiteres Ausführungsbeispiel mit Ringspalt zwischen Dilatator und Schleuse,
- Fig. 5: ein vorübergehendes Vorschieben des Dilatators zum Abreißen des Spitzenbereichs der Schleuse, und
- Fig. 6: das Zurückziehen des Dilatators inder Schleuse.

Die Einführvorrichtung ist generell in der Weise ausgebildet und ihre Benutzung erfolgt derart, wie dies in WO 02/43791 A1 angegeben ist. Die Schleuse wird vorzugsweise dazu benutzt, eine intravasale Blutpumpe in ein Blutgefäß einzubringen. Die Blutpumpe, die aus einem Antriebsteil und einem Pumpenteil besteht, hat generell zylindrische Form und einen Außendurchmesser von weniger als 4 mm.

Die Einführvorrichtung 10 zum Einbringen eines Gegenstandes, z.B. einer Blutpumpe, in ein Körpergefäß weist einen langgestreckten Dilatator 11 auf, der aus einem geraden Schaft mit zylindrischer glatter Oberfläche 12 besteht. Durch den Dilatator 11 erstreckt sich ein zentraler Kanal 13, in dem ein (nicht dargestellter) Führungsdraht verlaufen kann. Über den Führungsdraht wird der Dilatator 11 geschoben. An seinem distalen Ende weist der Dilatator 11 einen konischen Spitzenbereich 14 auf, mit dem er beim Vorschieben das Körpergewebe auseinander drückt und den entstehenden Körperkanal dilatiert. Der Dilatator 11 besteht aus starrem Kunststoff. Seine Wandstärke ist so groß, dass keine spürbaren Deformierungen möglich sind. Biegungen sind möglich, Knicke hingegen faktisch ausgeschlossen.

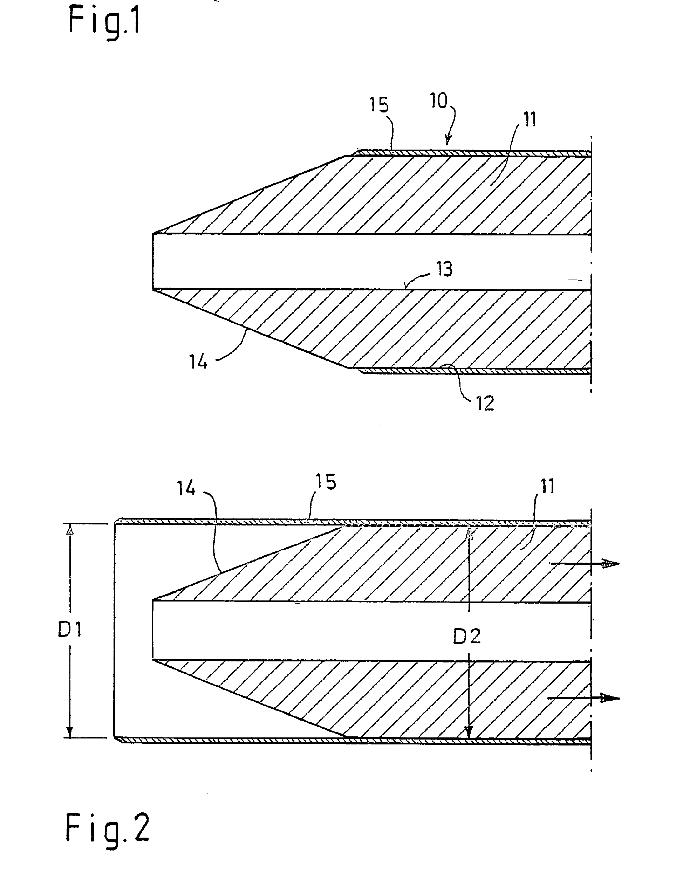

Auf den Dilatator 11 ist die Schleuse 15 aufgeschoben. Die Schleuse besteht aus einem rohrförmigen Mantel mit einer Wandstärke von maximal 0,06 mm, vorzugsweise von maximal 0,04 mm und insbesondere von etwa 0,03 mm. Das Material der Schleuse 15 ist harter Kunststoff, wie Polyamid oder Polyester. Die Schleuse 15 sitzt im Presssitz auf dem Dilatator 11. Dies bedeutet, dass der Innendurchmesser D1 der Schleuse 155 geringfügig kleiner ist als der Außendurchmesser D2 des Dilatators 11 (Figur 2).

Wenn der Dilatator in den Körper des Patienten eingeführt wird, liegt das distale Ende der Schleuse 15 hinter dem Spitzenbereich 14. Wenn das distale Ende der Schleuse 15 sich im Blutgefäß befindet, wird der Dilatator 11 gemäß Figur 2 zurückgezogen, wobei die Schleuse 15 festgehalten wird und an ihrem Platz im Körper verbleibt. Eine Repositionierung der Schleuse kann nur mit erneut eingeführtem Dilatator erfolgen.

Die Umfangsfläche 12 des Dilatators 11 kann mit einer reibungsarmen Beschichtung, z.B. aus Teflon oder einer hydrophilen Dünnschicht versehen sein.

Wegen der Materialhärte der Schleuse 15 und wegen des festen Sitzes der Schleuse auf den Dilatator 11 besteht nicht die Gefahr des Aufstauchens beim Hindurchschieben durch die Haut oder in den kollagenen Strukturen der Gefäße im Gewebe, weil die Schleuse 15 weder nach innen noch nach außen ausweichen kann.

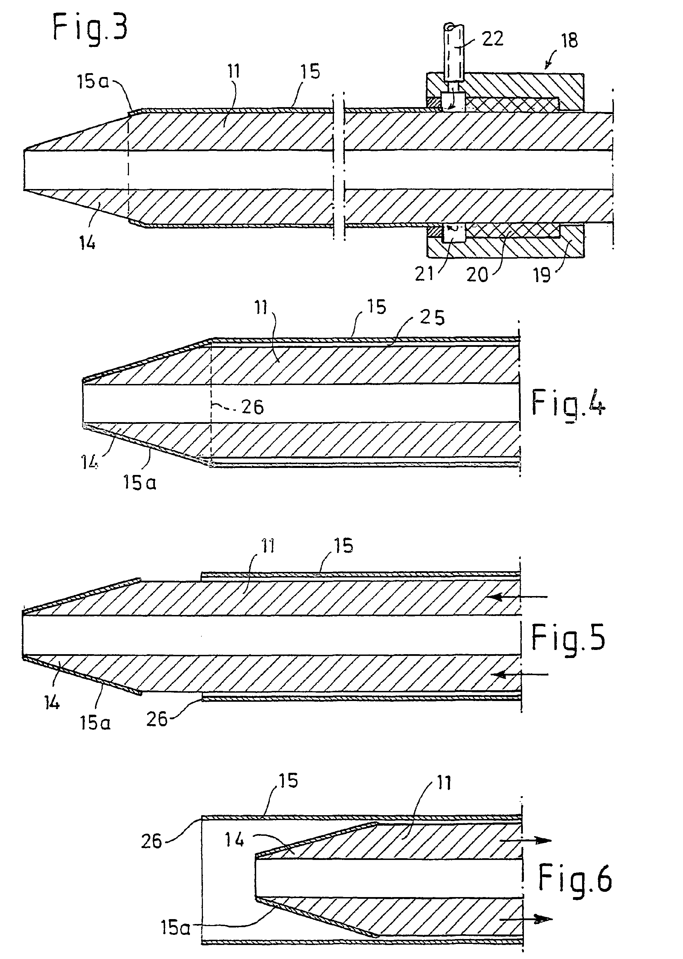

Figur 3 zeigt ein Ausführungsbeispiel, bei dem die Schleuse 15 einen distalen Endabschnitt 15a aufweist, der den konischen Spitzenbereich 14 des Dilatators teilweise überdeckt. Der Endabschnitt 15a bildet eine nach innen gerichtete Manschette der Schleuse, wodurch die Schleuse 15 gegen Zurückschieben auf dem Dilatator 11 gesichert ist.

Gemäß Figur 3 befindet sich an dem proximalen Ende der Schleuse 15 eine Anschlussvorrichtung 18, die hier nur schematisch dargestellt ist. Die Anschlussvorrichtung 18 weist ein Gehäuse 19 auf, welches das rückwärtige Ende der Schleuse 15 abdichtend umschließt und den Dilatator 11 durchlässt. Das Gehäuse 19 enthält ein hämostatisches Ventil 20, das nach dem Herausziehen des Dilatators 11 den Kanal durch die Schleuse 15 absperrt, so dass kein Blut austreten kann.

Außerdem enthält die Anschlussvorrichtung 18 innerhalb des Gehäuses 19 einen Ringkanal 21, der mit einem Zulaufrohr 22. verbunden ist, durch das Druckflüssigkeit zugeführt werden kann. Die Druckflüssigkeit tritt aus dem Ringkarial 21 in das rückwärtige Ende der Schleuse ein und sie bildet ein Gleitmittel, um den Dilatator 11 in der Schleuse 15 zurückziehen zu können.

Bei dem Ausführungsbeispiel von Figur 3 sitzt die Schleuse 15 im Passsitz auf dem Dilatator 11 und sie wird durch die Wirkung der Druckflüssigkeit geringfügig auseinandergedrückt, so dass sie abhebt und, sich ein dünner gleitfähiger Flüssigkeitsfilm zwischen Schleuse und Dilatator ausbildet.

In den Figuren 4-6 ist ein Ausführungsbeispiel dargestellt, bei dem der Innendurchmesser der Schleuse 15 etwas größer ist als der Außendurchmesser des Dilatators 11. Beispielsweise beträgt der Außendurchmesser des Dilatators 11 12 F (4,0 mm) und der Innendurchmesser der Schleuse 15 beträgt 13 F (4,33 mm). Der Ringspalt 25 ermöglicht ein reibungsarmes Zurückziehen des Dilatators 11. Auch bei diesem Ausführungsbeispiel beträgt die Wandstärke der Schleuse maximal 0,06 mm.

Die Schleuse 15 weist an ihrem proximalen Ende einen konischen Spitzenbereich 14 auf, der haftend mit dem Spitzenbereich 14 verbunden ist, z.B. durch Kleben. Der Endabschnitt 15a ist mit dem zylindrischen Bereich der Schleuse 15 durch eine umlaufende Abreißlinie 26 verbunden, beispielsweise eine Perforationslinie oder eine andere Schwächungslinie.

In der Konfiguration gemäß Figur 4 werden der Dilatator 11 und die Schleuse 15 über einem Führungsdraht in den Körper eingeführt. Wenn die Schleuse 15 in das Blutgefäß eingedrungen ist, wird zunächst gemäß Figur 5 der Dilatator 11 noch weiter vorgeschoben, so dass der Endabschnitt 15a an der Abreißlinie 26 von der Schleuse 15 abreißt. Der Endabschnitt 15a verbleibt auf dem Dilatator 11, der anschließend gemäß Figur 6 zurückgezogen wird, während die Schleuse 15 an ihrem Platz verbleibt.

## Patentansprüche

1. Einführvorrichtung zum Einführen eines Gegenstandes in ein Körpergefäß, mit einer rohrförmigen Schleuse (15) und einem die Schleuse (15) tragenden Dilatator (11), der einen konischen Spitzenbereich (14) aufweist und aus der Schleuse zurückziehbar ist,
**dadurch gekennzeichnet,**
**dass** die Schleuse (15) für den Durchgang einer intravasalen Blutpumpe bemessen ist und eine Wandstärke von maximal 0,06 mm hat und aus einem radial nicht dehnbaren harten Kunststoff besteht.

2. Einführvorrichtung nach Anspruch 1, **dadurch gekennzeichnet, dass** vor dem Aufschieben der Schleuse (15) auf den Dilatator (11) der Innendurchmesser (D1) der Schleuse (15) mindestens gleich groß ist wie der Außendurchmesser (D2) des Dilatators (11).

3. Einführvorrichtung nach Anspruch 2, **dadurch gekennzeichnet, dass** die Schleuse (15) in einem festen Sitz auf dem Dilatator (11) sitzt, derart, dass kein Spalt zwischen Dilatator und Schlauch vorhanden ist.

4. Einführvorrichtung nach einem der Ansprüche 1-3, **dadurch gekennzeichnet, dass** der Dilatator (11) und/oder die Schleuse (15) ein reibungsarmes Gleitmaterial aufweist.

5. Einführvorrichtung nach einem der Ansprüche 1-4, **dadurch gekennzeichnet, dass** eine Anschlussvorrichtung (18) zum Injizieren einer Druckflüssigkeit in die Schleuse (15) vorgesehen ist.

6. Einführvorrichtung, insbesondere nach einem der Ansprüche 1-5, **dadurch gekennzeichnet, dass** die Schleuse (15) einen distalen Endabschnitt (15a) aufweist, der den konischen Spitzenbereich (14) mindestens teilweise überdeckt.

7. Einführvorrichtung nach Anspruch 6, **dadurch gekennzeichnet, dass** der Endabschnitt (15a) durch eine Abreißlinie (26) begrenzt ist.

8. Einführvorrichtung nach einem der Ansprüche 1-7, **dadurch gekennzeichnet, dass** der Endabschnitt (15a) der Schleuse (15) haftend mit dem Spitzenbereich (14) des Dilatators (11) verbunden ist.

## Claims

1. An introduction device for introducing an object into a vessel of a body, comprising a tubular channel (15) and a dilator (11) carrying the channel (15), wherein the dilator (11) comprises a conical tip portion (14) and is adapted to be retracted from the channel,
**characterized in that**
the channel (15) is dimensioned for the passage of an intravascular blood pump and has a wall thickness not larger than 0.06 mm and is made from a hard plastic material that is not radially expandable.

2. The introduction device according to claim 1, **characterized in that** prior to sliding the channel (15) onto the dilator (11) the inner diameter (D1) of the channel (15) is at least as large as the outer diameter (D2) of the dilator (11).

3. The introduction device according to claim 2, **characterized in that** the channel (15) forms a force fit on the dilator (11) such that no gap exists between the dilator and the hose.

4. The introduction device according to one of claims 1-3, **characterized in that** the dilator (11) and/or the channel (15) comprise a low-friction slip material.

5. The introduction device according to one of claims 1-4, **characterized in that** a connecting device (18) for injecting a pressurized fluid into the channel (15) is provided.

6. The introduction device, in particular according to one of claims 1-5, **characterized in that** the channel (15) comprises a distal end section (15a) which at least partly overlaps the conical tip portion (14).

7. The introduction device according to claim 6, **characterized in that** the end section (15a) is defined by a tear-off line (26).

8. The introduction device according to one of claims 1-7, **characterized in that** the end section (15a) of the channel (15) is adhesively bonded to the tip portion (14) of the dilator (11).

## Revendications

1. Dispositif d'introduction permettant d'introduire un objet dans un vaisseau corporel, comprenant un sas (15) de forme tubulaire et un dilatateur (11) portant le sas (15), dilatateur qui présente une région en pointe (14) conique et peut être retiré du sas,
**caractérisé en ce que**
le sas (15) est dimensionné pour le passage d'une pompe à sang intravasculaire et possède une épaisseur de paroi de 0,06 mm, au maximum, et est réalisé en une matière plastique dure, non extensible radialement.

2. Dispositif d'introduction selon la revendication 1, **caractérisé en ce qu'**avant l'enfilage du sas (15) sur le dilatateur (11), le diamètre intérieur (D1) du sas (15) est au moins aussi grand que le diamètre extérieur (D2) du dilatateur (11).

3. Dispositif d'introduction selon la revendication 2, **caractérisé en ce que** le sas (15) est placé selon un ajustement serré sur le dilatateur (11), de telle manière qu'aucun interstice ne soit présent entre le dilatateur et le sas.

4. Dispositif d'introduction selon l'une des revendications 1 à 3, **caractérisé en ce que** le dilatateur (11) et/ou le sas (15) présente un matériau glissant à faible frottement.

5. Dispositif d'introduction selon l'une des revendications 1 à 4, **caractérisé en ce qu'**un dispositif de raccordement (18) est prévu pour l'injection d'un liquide sous pression dans le sas (15).

6. Dispositif d'introduction, en particulier selon l'une des revendications 1 à 5, **caractérisé en ce que** le sas (15) présente un tronçon d'extrémité (15a) distal, qui recouvre, au moins partiellement, la région en pointe (14) conique.

7. Dispositif d'introduction selon la revendication 6, **caractérisé en ce que** le tronçon d'extrémité (15a) est délimité par une ligne de rupture (26).

8. Dispositif d'introduction selon l'une des revendications 1 à 7, **caractérisé en ce que** le tronçon d'extrémité (15a) du sas (15) est relié par adhérence à la région en pointe (14) du dilatateur (11).
